# EUROPEAN PATENT APPLICATION

(11) **EP 1 431 902 A2**
(43) Date of publication of application: **23.06.2004**
(21) Application number: 03258134.0
(22) Date of filing: 22.12.2003
(51) Int. Cl.: G06F 19/00, G06F 17/60

(54) **System and method for prescription management and delivery**

(30) Priority: 20.12.2002 US 326245; 20.12.2002 US 326431
(71) Applicant: PDX, Inc., Fort Worth, Texas 76108-2253 (US)
(72) Inventor: Hill, Sr. Kenneth A., Texas 76107 (US); Stalzer, Mark A., Texas 76137 (US); Bloodgood, Sean P., Texas 76109 (US); Crosslin, Todd A., Texas 76017 (US); McCray, John T., Texas 76048 (US)
(74) Representative: Lawrence, John

(57) **Abstract**

A method and system for prescription management is described. At least one prescription is received at a pharmacy and an order associated with at least one of the prescriptions is generated. A shipping location associated with the order is determined and the order is communicated to a central fill facility for filling the prescriptions associated with the order. A cost associated with the order is received from the central fill facility. The cost includes a shipping cost and an order cost. Payment is settled for the order based on the cost. In one embodiment, the prescription is filled with the prescribed drug at the central fill or a remote third-party facility for delivery of the drug to a location designated by the patient on behalf of, but independent of, the designated pharmacy.

## Description

### BACKGROUND OF THE INVENTION

As computers have grown increasingly important in today's society, businesses and consumers in the medical field have also increasingly used computers for a variety of tasks. Existing systems have provided the capability to fill prescriptions for a plurality of pharmacies at a central location remote from the pharmacies. The central filing system allows pharmacies to fill prescriptions using an automated system as opposed to standard manual filling of the prescription at the pharmacy itself which may result in cost savings for the pharmacies. The central filling system then sends the filled prescriptions back to the pharmacy for verification by the pharmacist and purchase by the patient.

### SUMMARY OF THE INVENTION

According to one embodiment of the present invention, a method and system for prescription management is described. At least one prescription is received at a pharmacy and an order associated with at least one of the prescriptions is generated. A shipping location associated with the order is determined and the order is communicated to a central fill facility for filling the prescriptions associated with the order. A cost associated with the order is received from the central fill facility. The cost includes a shipping cost and an order cost. Payment is settled for the order based on the cost.

According to another embodiment to another embodiment of the present invention, a method for prescription fulfillment is described. A prescription of a prescribed drug for a patient is received at a pharmacy designated by the patient. While maintaining the prescription at the designated pharmacy, the prescription is filled with the prescribed drug at a remote third-party facility for delivery of the drug to a location designated by the patient on behalf of, but independent of, the designated pharmacy.

The present invention provides a number of technical advantages over previous techniques. Various embodiments of the present invention may provide some, all or none of these technical advantages. For example, the automated prescription processing system can efficiently process and fill an extremely large volume of prescription requests from a central fill inventory using robotics. As a result, the productivity of pharmacy professionals can be increased, and customers' waiting times for prescriptions to be filled can be decreased. Also, as a result of using a central fill inventory to fill prescription requests (e.g., in government-funded indigent patient, long-term care programs), the government-funded inventory is not merged in any respect with local pharmacies' inventories. Also, pharmaceutical suppliers may maintain a centralized inventory of drugs in a manner that takes full advantage of economies of scale to minimize costs. Through freeing up pharmacists' time by filling prescriptions with high-speed robotics at remote sites, rather than having pharmacists fill them manually, the quality of the long-term patient care and services being provided may be improved, and the costs for providing that level of care and those services may be decreased.

Another technical advantage of one or more embodiments is providing home delivery of filled prescriptions. For example, a local pharmacy chosen by the patient may receive, review, process and maintain a prescription which may be filled at a centralized or other facility. The fulfillment facility may deliver the filled prescription to the pharmacists or directly to the patient as directed by the pharmacist and the patient. Refills may be similarly processed and may be ordered at the pharmacy or electronically through an automated system or electronic site associated with the pharmacy. Thus, the pharmacy selected by and/or local to the patient may renew and maintain prescriptions of these patients to ensure proper care is provided to the patient while off-loading the actual fill, which is expensive and time consuming, and does not require the pharmacist's expertise. As a result, the pharmacist may spend more time with the patients, and in determining how to best fill a prescription and manage the needs of the patient. In addition, home delivery provides greater convenience for the patient, who may have trouble getting to the pharmacy or otherwise have to rely on others. Thus, independence of patients may be improved.

Other technical advantages of the present invention will be readily apparent to one skilled in the art from the following figures, description and claims.

### BRIEF DESCRIPTION OF THE DRAWINGS

For a more complete understanding of the present invention and its advantages, reference is now made to the following descriptions, taken in conjunction with the accompanying drawings, in which:
FIGURE 1 is a block diagram that illustrates a prescription filling system according to one embodiment of the present invention;
FIGURE 2 is a block diagram illustrating an order associated with the prescription filling system of FIGURE 1 according to one embodiment of the present invention;
FIGURES 3A and 3B are a flow diagram illustrating a method for filling prescriptions according to one embodiment of the present invention;
FIGURE 4 is a flow diagram illustrating a method for communicating prescription request information according to one embodiment of the present invention;
FIGURES 5A and 5B are a flow diagram illustrating a method for handling prescription filling according to one embodiment of the present invention;
FIGURES 6A and 6B are a flow diagram illustrating a method for filling prescriptions according to one embodiment of the present invention;
FIGURE 7 is a flow diagram illustrating a method of operation of a central fill system according to one embodiment of the present invention;
FIGURE 8 is a flowchart illustrating handling of the orders according to one embodiment of the present invention; and
FIGURE 9 is a flowchart illustrating further details of payment processing for prescription according to one embodiment of the present invention.

### DETAILED DESCRIPTION OF THE INVENTION

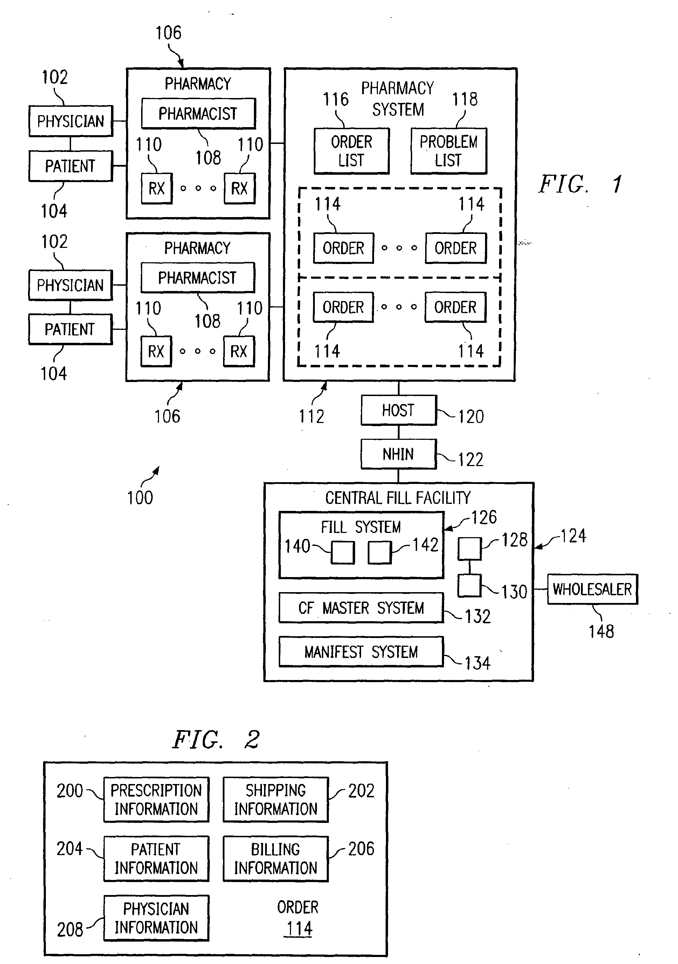

FIGURE 1 is a block diagram illustrating a home delivery system 100 according to one embodiment of the present invention. System 100 comprises one or more physicians 102, one or more patients 104, one or more pharmacies 106, a pharmacy system 112, a host 120, a National Health Information Network (NHIN®) 122 and a central fill facility 124. In general, home delivery system 100 supports home delivery of filled prescriptions to patients and provides increased functionality to pharmacists. Physicians 102 and patients 104 interact with pharmacy system 112 and central fill facility 124 to fill prescriptions. Filled prescriptions may be delivered by central fill facility 124 to pharmacies 106 for purchase by patient 104. Filled prescriptions may also be home delivered by central fill system 126 or an intermediate shipper directly to patient 104.

Physician 102 comprises any suitable prescriber of pharmaceuticals. Physician 102 may write a prescription with or without refills for patient 104.

Patient 104 comprises a consumer of pharmaceuticals, such as a human, animal or other suitable entity. Patient 104 may or may not have an existing relationship with physician 102, pharmacy 106 and/or a pharmacist and generally comprises one who receives one or more prescriptions.

Each pharmacy 106 comprises any suitable provider of pharmaceuticals. The pharmacies may be part of a pharmacy chain and/or independent of each other. For example, a particular pharmacy 106 may comprise a pharmacy local to patient 104 and familiar with patient 104.

Pharmacies 106 are associated with a pharmacist 108 and one or more prescriptions 110. Pharmacist 108 comprises a pharmacist or other professional licensed to dispense pharmaceuticals to patients 104 based on prescriptions 110. Pharmacist 108 may be associated with pharmacy 106 and pharmacy system 112.

Prescription 110 comprises an electronic, handwritten, or other suitable form of pharmaceutical prescription for one or more pharmaceuticals. Prescription 110 is associated with patient 104 for whom the prescription is written, physician 102 who writes the prescription and pharmacy 106 which owns, maintains and processes the prescription. For example, pharmacy 106 associated with a particular prescription 110 may be a pharmacy local to patient 104 which may have developed a relationship with patient 104, such as by knowing at least a portion of the medical history of patient 104, and provides increased quality of service based on the historical knowledge.

Pharmacy system 112 comprises hardware and/or software, such as software stored on a computer readable medium and loadable into a memory associated with a processor for execution, operable to support handling of one or more prescriptions 110. Pharmacy system 112 is associated with one or more pharmacies 106. In one embodiment, pharmacy system 112 may comprise a computer-implemented system for processing prescriptions 110 and one or more pharmacies 106 associated with pharmacy system 112. As such, the functions of pharmacy system 112 can be implemented by one or more application programs executed in memory (not shown) by an appropriate computer processor (not shown). The application programs may be distributed programs with a portion running at each pharmacy 106 and a portion running at a central server or site for the pharmacy system 112. Alternatively, for example, each pharmacy may run respective computer implemented process for processing prescriptions 110.

In one embodiment, pharmacy system 112 comprises one or more orders 114, an order list 116 and a problem list 118. Each order 114 comprises one or more prescriptions 110 for filling by central fill facility 124. Prescriptions 110 may be grouped together into order 114 according to suitable criteria by pharmacist 108 at pharmacy 106 which owns prescriptions 110. For example, order 114 may include several prescriptions to be filled for a single family, a nursing home or multiple individuals. In general, a pharmacy owns a prescription by keeping title to the prescription even if the prescription is being filled at a different and/or unrelated facility.

Order list 116 comprises one or more orders 114. Orders 114 in order list 116 may be sorted by date and time of entry. Orders 114 may also be sorted by a priority 2016 associated with orders 114. Priority 2016 may be used by pharmacy system 112 to send higher priority orders to central fill facility 124 sooner than lower priority orders. For example, a rush order may have a higher priority than an ordinary refill. In general, orders 114 may be sorted by suitable criteria in suitable combination in order list 116. For example, orders 114 may be sorted first by priority and then by date and time of entry.

Referring to FIGURE 2, one embodiment of order 114 is illustrated. In this embodiment, each order 114 comprises one or more sets of prescription information 200 which are grouped together for filling by central fill facility 124, shipping information 202 for the filled prescriptions, patient information 204 associated with one or more patients 104 associated with prescriptions 110 grouped into order 114, billing information 206 associated with order 114 and physician information 208 associated with one or more physicians who issued the grouped prescriptions 110.

Prescription information 200 comprises a set of data associated with an electronic, handwritten, or other suitable form of pharmaceutical prescription for one or more pharmaceuticals, such as prescription 110, entered into pharmacy system 112. For example, prescription information 200 may comprise the number of refills remaining, the total number of refills authorized, the name of the patient associated with the prescription, the issuer of the prescription, whether generic equivalents are allowed and the name of the prescribed pharmaceutical. Sets of prescription information 200 associated with prescriptions 110 may be grouped together into a single order 114.

Shipping information 202 comprises at least one of a shipping location, shipping cost, delivery date and other suitable information associated with delivering a filled order 114. Shipping information 202 may be used to indicate where a filled order 114 is to be sent after filling at central fill facility 124. For example, home delivery of a filled order 114 may use shipping information 202 to indicate to central fill facility 124 that a filled order 114 is to be home delivered directly to a patient's home from central fill facility 124.

Billing information 206 comprises data associated with how prescription 110 or order 114 will be paid for. For example, billing information 206 may comprise suitable information for authorizing and charging a credit card or debit card, check payment, insurance payment and other third-party payment techniques. Billing information 206 may handle multiple payment methods for a single prescription 110 or order 114, such as payment by an insurance carrier and credit card.

Physician information 208 comprises data associated with a physician which has issued prescription 110. For example, physician information 208 may include contact information for the physician, state(s) of licensure for the physician and other suitable data. Physician information 208 may be maintained on a prescription-by-prescription basis, using relational database techniques, such as a central database of physician information with is linked or associated with prescriptions 110 as appropriate, or using other suitable techniques. For example, a central listing of physician information 208 for all physicians 102 which have issued prescriptions 110 that have been filled at pharmacy 106 may be maintained and the appropriate physician information 208 may then be associated with prescription information 200 for new prescriptions 110 entered into pharmacy system 112.

Patient information 204 comprises data associated with patient 104. For example, patient information 204 may comprise one or more names for the patient, such as married name and maiden name, an address, one or more phone numbers, medical history and other suitable patient-related information. In general, patient information 204 comprises data usable by pharmacist 108 to service patient 104. For example, a particular pharmacist may track prior diagnosis and counseling notes in patient information 204.

Returning to FIGURE 1, problem list 118 comprises a list of one or more problems detected by pharmacist 108 with order 114 maintained by pharmacy system 112. For example, an impractically or illegally large dosage of a particular pharmaceutical may be listed in a prescription. For another example, a billing problem may have been found with billing information 206 associated with order 114. For example, a problem may be resolved by contacting any of the patient, physician and the insurer associated with an order 114. In one embodiment, a priority may be associated with each problem in problem list 118 indicating the importance and/or urgency of the problem. Problem list 118 may be sorted by priority, date and time of entry of the problem or by other suitable criteria.

Host 120 comprises software and/or hardware, alone or in suitable combination, operable to communicate information between pharmacy system 112 and NHIN 122. More specifically, host 120 is operable to route and translate information between pharmacy system 112 and NHIN 122. Pharmacy system 112 used by a particular group of pharmacies 106 may maintain data in a format which is incompatible with NHIN 122 and central fill system 126. Host 120 provides the capability to translate between the data formats and route information from pharmacy system 112 to NHIN 122.

In one embodiment, host 120 may be further operable to receive inventory pricing and prescription status from NHIN 122 on a store-by-store basis. More specifically, host 120 may maintain pricing information for pharmaceuticals for pharmacies 106 in a particular pharmacy system 112. The pricing information may be retrieved from NHIN 122 and stored on a pharmacy-by-pharmacy basis. For example, if the price from wholesaler 148 for aspirin is $1 per bottle, a first pharmacy associated with pharmacy system 112 may charge $1.50 per bottle for aspirin and a second pharmacy ' associated with pharmacy system 112 may charge $1.75 per bottle for aspirin. Host 120 is operable to store the prices and/or price difference for each respective store in pharmacy system 112. Prescription status may be maintained by host 120 to allow pharmacies 106 to determine whether a prescription sent to central fill system 126 has been filled, the expected date of fill for an unfilled prescription, the date of fill for a filled prescription and other suitable information.

In one embodiment, host system 120 may comprise a PDX Host System®, which is offered by PDX Inc. as a database maintenance, administrative and communications application for pharmacy systems. In this embodiment, host system 120 may further use a Unix Tunnel Daemon (UTD), which comprises a suitable combination of software and/or hardware operable to transfer large data files between system or network nodes. For example, in a Unix operating system context, daemons comprise programs or processes that typically run in the background and attend to various tasks. For this example embodiment, a UTD may be used to perform the task of transferring data files containing prescription information between different system nodes. In general, host system 120 may comprise suitable data communications capabilities operable to perform data transfer functions. In one embodiment, UTD comprises a daemon program operable to route data between one or more recipients and one or more senders.

Alternatively, a Pass-through Tunnel Daemon (PTD) may be used in place of, or in suitable combination with, the UTD. The PTD comprises software operable to forward data between a host computer and the NHIN, and configurably log appropriate data, such as a NHIN account number, a user or store identifier, a prescription number and/or a transaction number.

NHIN 122 may further comprise a data center that provides pharmacies and other customers in the pharmaceutical industry with a comprehensive database of up-to-date and standardized drug file information, and maintenance of third party files. For example, an NHIN database may maintain the following data files: drug files; third party plan benefit files; SIG files including, for example, dosing instructions; physician files; drug manufacturer information files; and disease management files. For this embodiment, NHIN system 122 also includes a UTD for routing prescription request information to other system nodes.

Further, NHIN 122 may communicate with multiple hosts 120 respectively associated with distinct pharmacy systems 112. For example, a pharmacy system associated with one pharmacy chain may communicate with NHIN 122 through a first host and a second pharmacy chain may communicate with NHIN 122 through a second host.

Also, NHIN 122 may be operable to route data from hosts 120 to central fill system 126. For example, hosts 120 may use infrastructure associated with NHIN 122 to communicate information from pharmacy system 112 to central fill system 126.

In addition, NHIN 122 may be further operable to maintain pharmaceutical pricing and inventory information. NHIN 122 may receive and store pharmaceutical pricing information from wholesalers 148 for use by hosts 120. NHIN 122 may also receive and store inventory status on pharmaceuticals from wholesalers 148 for use by hosts 120. For example, a first wholesaler may have 1000 aspirin pills currently in stock, while a second wholesaler may have 5000 aspirin pills currently in stock.

Central fill facility 124 comprises software and/or hardware, either alone or in suitable combination, operable to manage filling of orders 114 and prescriptions 110. More specifically, central fill facility 124 comprises a central fill system 126, a processor 128, storage 130, a central fill master system 132, and a manifest system 134.

In one embodiment, central fill system 126 receives information related to prescription 110 from pharmacy system 112 and fills prescription 110. Central fill system 126 then prepares the filled prescriptions for delivery to pharmacy 106 which owns prescriptions 110 filled by central fill system 126.

In one embodiment, central fill system 126 may comprise a central fill administrative system part 140 and an automated prescription drug dispensing system part 142. Drug dispensing system part 142 may have an inventory of one or more pharmaceuticals. In one embodiment, multiple central fill facilities may be a part of central fill system 126. In one embodiment, dispensing system part 142 maintains a central fill inventory of drugs manufactured and/or sold by one or more pharmaceutical wholesalers 148.

Processor 128 comprises any suitable general purpose or specialized electronic processing device, such as a central processing unit (CPU), operable to communicate with storage 130 and execute software. Storage 130 comprises any suitable combination of transient or persistent optical, magnetic, electronic or other computer readable storage medium such as a floppy disk drive, a hard disk drive, a CD-ROM drive, a CD-RW drive, a magnetic tape drive, an optical drive, random access memory (RAM), static RAM (SRAM) and read-only memory (ROM). Storage 130 may also represent multiple computer readable storage devices.

Central fill master system 132 comprises software and/or hardware, either alone or in suitable combination, such as software stored on storage 130 and executable by processor 128, operable to manage central fill system 126 and manifest system 134 to fill orders 114 and deliver the filled orders.

Manifest system 206 comprises software and/or hardware, either alone or in suitable combination, operable to determine shipping costs, generate shipping labels, determine shipping method and determine shipping location of a filled order 114 and/or a filled prescription 110. For example, manifest system 206 may determine that United Parcel Service (UPS) shipping is cheaper to a given location at a given time than U.S. Postal Service delivery. Filled prescriptions may be delivered to pharmacy system 112, pharmacies 106 or a patient's designed address.

Wholesalers 148 comprise businesses or other suitable entities operable to maintain raw materials and pharmaceuticals useable by central fill system 126 for filling prescriptions 110. For example, a wholesaler may comprise a company such as Dow Chemical which supplies pharmaceuticals to central fill facilities for use in filling prescriptions 110.

In operation, patient 104 may bring one or more prescriptions 110 to pharmacist 108 at pharmacy 106. Prescriptions 110 are then entered into pharmacy system 112. Alternatively, physician 102 may use an automated prescription handling system to convey a new prescription via a communication link to pharmacy system 112. In one embodiment, the electronic transmission of prescription information between perscribers and providers is performed in accordance with a current National Council for Prescription Drug Programs (NCPDP) Script Standard (e.g., Script Standard V.1.5). This standard addresses, among other things, the electronic transmission of new prescriptions, prescription refill requests, prescription fill status notifications, and prescription cancellation notifications.

In general, one or more sets of prescription information 200 respectively associated with prescriptions 110 are entered into pharmacy system 112. In one embodiment, related patient information 204, physician information 208 and billing information 206 may be associated with prescription information 200 to allow, for example, more convenient access to related information by pharmacist 108.

Prescription 110 may be entered into pharmacy system 112 in a number of different ways. For example, a pharmacy employee or technician can use a graphical user interface (GUI) to enter a new or refill prescription 110 received from a patient 104, physician 102, or other suitable source into pharmacy system 112, by typing in the prescription information to a prescription filling screen (e.g., Multiple Fill Queue screen for refills, or Rx Filling screen for new prescriptions) displayed on a computer monitor. Alternatively, for example, patient 104 may enter a request for a prescription refill via the Internet (e.g., using a browser to access the pharmacy system's web page and enter prescription refill information). Also, as another example, patient 104 can use an Interactive Voice Response (IVR) system associated with pharmacy system 112 to enter a request for a prescription refill. The IVR system allows a patient to ask questions and provide answers for pharmacy system 112 by pressing the appropriate keys on a touch-tone phone, or by stating certain words such as "yes" or "no".

Pharmacist 108 then generates an order 114 for one or more prescriptions 110 owned by pharmacy 106. More specifically, pharmacist 108 may group one or more sets of prescription information 200 for filling at central fill facility 124. Pharmacist 108 may group sets of prescription information 200 into order 114 regardless of the contents of prescription information 200. For example, an order 114 may include prescriptions regardless of the name on the prescriptions, such as a mother and child with different last names. For another example, an order 114 may include many prescriptions from many different patients 104, such as a group of patients at a nursing home.

Pharmacist 108 may also determine at least a portion of shipping information 202 for order 114. Based on instructions from patient 104, practical considerations, store policy and/or other suitable criteria, pharmacist 108 determines where the filled order 114 is to be delivered. For example, order 114 may be home delivered to a nursing home or a patient's home, or may be delivered to pharmacy 106 for distribution by pharmacist 108. In one embodiment, order 114 may have different shipping locations for different portions of order 114.

Pharmacist 108 may next perform a Drug Utilization Review (DUR) using prescription information 200 and associated patient information 204 and physician information 208. For example, pharmacist 108 may review past and present medical and/or drug history associated with patient 104, such as data stored in patient information 204, and determine if harmful drug interactions may occur. For another example, a pharmacist with a long-standing relationship with patient 104 may use the pharmacist's historical knowledge and understanding of the patient while performing the DUR.

If shipping information 202 indicates that order 114 is to be delivered to pharmacy 106, then order 114 is communicated to central fill facility 124 for filling. At central fill facility 124, order 114 is managed by master system 132, filled by central fill system 126, and delivered back to pharmacy 106. Pharmacist 108 may then perform a quality assurance review of the filled prescriptions 110 in order 114 to check, for example, that proper pharmaceuticals have been dispensed in the proper dosages.

If shipping information 202 indicates home delivery of order 114, either in whole or in part, then pharmacist 108 may determine payment information for order 114. Pharmacy system 112 may estimate an order cost for order 114. For example, pharmacy system 112 may use pricing information available at host 120 to determine an estimated order cost for the prescriptions 110 in order 114. For example, if the payment technique for order 114 is by credit card, then pharmacy system 112 may authorize, but not actually charge, the credit card to determine whether the credit card has sufficient capacity to pay for order 114. For another example, if the payment technique is by check, pharmacy system 112 may determine whether sufficient funds are available to cover the check. For yet another example, if payment is via an insurance carrier or government organization, then pharmacy system 112 may determine whether order 114 will be covered. In general, pharmacy system 112 may take appropriate action to determine whether payment can be made on order 114.

Next, pharmacist 108 may review order 114 for correct entry of prescription information 200 from prescription 110. Pharmacist 108 may also perform other suitable checks and verifications on order 114. Order 114 is then communicated to central fill facility 124 through host 120 and NHIN 122. In one embodiment, an identifier associated with order 114 is communicated to central fill master system 132 and central fill master system 132 uses the identifier to access order 114 store at pharmacy system 112. Alternatively, a copy of order 114 may be communicated to central fill master system 132, or a copy of one or more portions of order 114 may be communicated to central fill master system 132 with other portions remaining at pharmacy system 112 and accessible by central fill master system 132 using suitable techniques.

At central fill facility 124, master system 132 receives orders 114 and manages the filling of orders 114. More specifically, master system 132 organizes orders 114, such as by the particular prescription information 200 in order 114, and communicates orders 114 and/or portions of orders 114 to central fill system 126 for filling. For example, central fill system 126 may control a robotic pharmaceutical filling system. Central fill system 126 then labels the container for the filled prescription to indicate the name of the pharmaceutical and other suitable information. In one embodiment, central fill system 126 may include the capability to generate a label for the container for the filled prescription that include suitable patient information from patient information 204 and identifying information associated with pharmacy 106, such as a logo, as well as information associated with the filled prescription.

A pharmacist at central fill facility 124 then examines filled prescriptions to verify that the proper dosage of the proper pharmaceutical has been placed in the prescription container and that the label is correct. For example, the pharmacist at central fill facility 124 may comprise a professional with similar training and licensing to pharmacist 108. The pharmacist at central fill facility 124 may also perform other suitable verification checks.

Once the fill of the prescriptions has been verified, master system 132 passes order 114 to manifest system 134 to handle delivery. Manifest system 134 then determines a weight for the filled order 114 and packages the filled order 114 appropriately. For example, orders 114 involving containers holding liquids may involve different packaging issues than containers holding only solids. Manifest system 134 next determines a shipping cost associated with shipping and handling order 114. The shipping cost may include special handling provisions such as refrigeration or rush deliveries. Manifest system 134 may also determine which shipping method to use. For example, if the shipping cost to a particular location by a particular time is cheaper on UPS than the U.S. Postal Service, then manifest system 134 may select UPS for a particular order 114. Manifest system 134 then communicates the shipping cost information to master system 132.

Manifest system 134 may also sort multiple orders 114 by shipper. For example, manifest system 134 may direct filled orders 114 to be shipped by UPS to a first specific area, while U.S. Postal Service shipped orders 114 are directed to a second specific area. By sorting packages based on shipper, increased efficiency and capacity may be achieved by central fill facility 124. Manifest system 134 then home delivers the filled order 114 directly to the destination, such as the home of patient 204, indicated in shipping information 202.

Master system 132 receives the shipping cost information from manifest system 134. Master system 132 may then update the shipping information 202 associated with order 114 to indicate, for example, date of shipment, expected date of delivery and cost of shipping. In one embodiment, master system 132 communicates the updated shipping information 202 to NHIN 122 for distribution to pharmacy system 112 through host 120. Master system 132 may then communicate order 114 back to pharmacy system 112 with a status indication that order 114 has been filled.

Pharmacy system 112 may then finally settle the order 114 using the payment technique indicated in billing information 206. For example, a credit card may be actually charged or a check cashed.

In one embodiment, system 100 may allow pharmacies to enjoy economies of scale in pricing prescriptions by providing access to a central fill facility shared by many pharmacies. For example, independent pharmacies may not generate sufficient volume to receive discounted pricing on pharmaceuticals from wholesalers; however, by sharing the large capacity of the central fill facility across many small, medium and large pharmacy chains, the central fill facility is able to purchase pharmaceuticals from wholesalers at reduced rates and pass on these cost savings to pharmacies which use the central fill facility.

Also, in one embodiment, system 100 may allow local pharmacies to provide increased counseling and patient care while also providing the cost advantages that may be associated with mail order pharmacies. The mail order pharmacies often have decreased overhead from not maintaining a physical retail location, but are unable to. provide personalized service to the patient because contact with the patient is often through the mail and over the phone. The local pharmacies often provide increased patient counseling and more personalized service, but the prescriptions may cost more at the local pharmacy. System 100 may allow pharmacies of various sizes to provide both lower cost and personalized service to patients. Further, as system 100 supports home delivery of filled prescriptions, system 100 may also provide increased convenience to patients and pharmacists by supporting delivery of the filled prescriptions directly to the patient's residence.

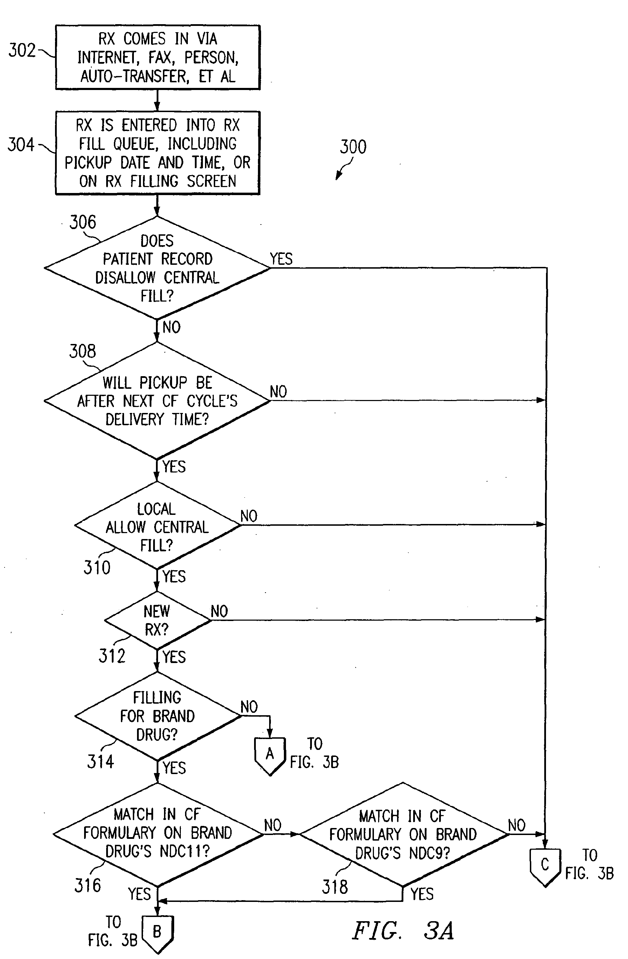

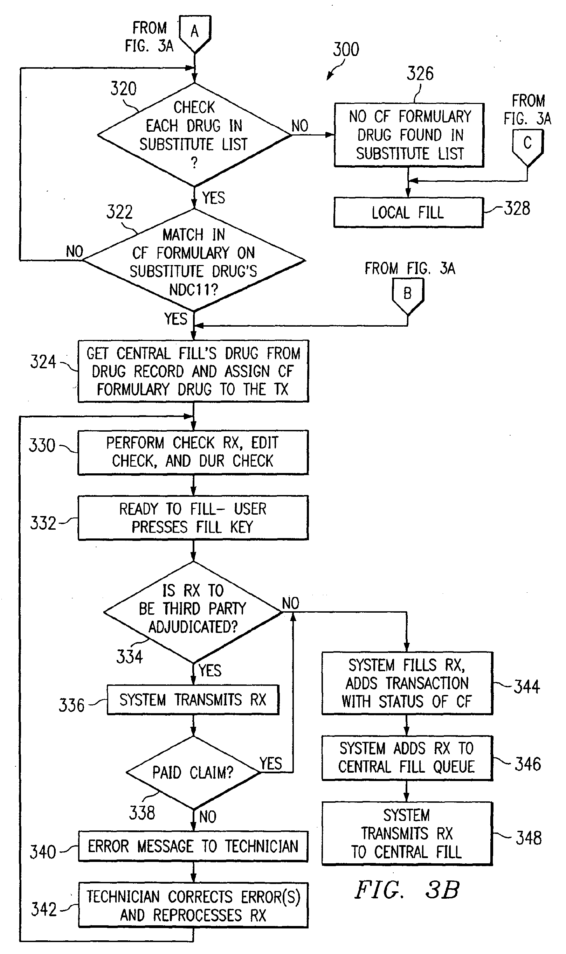

FIGURES 3A and 3B are a flow diagram illustrating an exemplary method 300 for filling prescriptions according to one embodiment of the present invention. At step 302, a request for a new or refill prescription from a patient or prescriber is received by pharmacy system 112. As mentioned above, the request can be received via a number of different ways, such as, for example, via the Internet, facsimile, electronic transfer of data, in person from the patient, or any other appropriate way of conveying prescription information.

At step 304, the received prescription information is entered, including pickup date, time, and payment information (if prescription is to be home delivered to the patient). For example, information may be entered into an "RX Fill Queue" screen or "Rx Filling" screen displayed on a computer monitor. Alternatively, information already in electronic form may be automatically accepted into a program, such as an electronic prescription.

At step 306, a central fill eligibility check is initiated by pharmacy system 112 determining whether the patient's record includes information that prohibits the prescription from being filled by a central fill facility. If the patient record shows that the prescription may be filled by a central fill facility, at step 308, pharmacy system 112 determines whether the prescription pickup date and time selected by the patient is subsequent to the central fill system's next scheduled delivery date and time (if prescription is to be home delivered, delivery date and time may not have to be determined as the home delivered prescription may be ordered several days before the patient has need of the prescription). If so, at step 310, pharmacy system 112 determines whether the local government (e.g., State where pharmacy store is located) allows the specific drug schedule to be filled by a central fill facility. For this embodiment, a flag specified for that function can be set. If the local government allows the drug schedule to be filled by a central fill facility, at step 312, pharmacy system 112 determines whether the prescription to be filled is for a new prescription. If so, pharmacy system 112 then determines whether the local government allows new prescriptions to be filled by a central fill facility (again, for example, by determining whether a flag specified for that function is set). If new prescriptions may be filled by a central fill facility, pharmacy system 112 proceeds to step 314.

Returning to step 306, if pharmacy system 112 determines that patient 104 does not want a central fill facility to be used, then at step 328, the pharmacy system initiates a procedure to fill the prescription request from the pharmacy system's local inventory. This procedure is also initiated (step 328) if pharmacy system 112 determines (1) that the prescription pickup date and time selected by the patient is prior to the central fill system's next scheduled delivery date and time (step 308), (2) the local State government does not allow the specific drug schedule to be filled by a central fill facility (step 310), and/or (3) the current prescription is new and the local State government does not allow new prescriptions to be filled by a central fill facility (step 312).

If central fill is allowed by the patient and the local State government, then at step 314, pharmacy system 112 next determines whether the pending prescription request includes a "brand name" drug. If so, at step 316, pharmacy system 112 searches a database including a formulary available from central fill system 126, in order to determine whether the "brand name" drug's name, manufacturer, strength, dosage form (such as pill, tablet or other suitable form) and package size (referred to as National Drug Code or NDC 11) matches the name, manufacturer, strength, dosage form and package size (NDC 11) of a drug listed in the central fill system's formulary. If a complete match is not found, at step 318, pharmacy system 112 searches the database with the central fill system's formulary of available drugs, in order to determine whether the "brand name" drug's name, manufacturer, dosage form and strength (referred to as NDC 9) matches the name, manufacturer, and strength (NDC 9) of a drug listed in the central fill system's formulary. If not, then pharmacy system 112 proceeds to step 328 to initiate a procedure to fill the request from the local pharmacy inventory. Otherwise, if a match is found in the central fill system's formulary at step 316 or step 318, then the pharmacy system 112 proceeds to step 324.

Returning to step 314, if the prescription request is not for a "brand name" drug, at step 320, pharmacy system 112 may search a database for the identity of any substitute drug authorized to fill the prescription request. At step 322, pharmacy system 112 can also determine from the database search whether an authorized substitute drug matches a drug listed in the central fill system's formulary of available drugs. If no drug on the substitute list matches any drug on the central fill system's formulary, then at step 328, pharmacy system 112 initiates the local fill procedure. However, if a substitute drug is found on the central fill system's formulary, step 322 leads to step 324. At step 324, if a substitute drug is used, pharmacy system 112 obtains from the database the pertinent central fill information that can be associated with the substitute drug, and assigns the appropriate drug in the central fill system's formulary as the substitute drug for the pending prescription request transaction. If a brand name drug is used, steps 314, 316 and 318 lead to step 324.

Once a drug from the central fill system's 126 formulary has been identified as available for processing, at step 330, pharmacy system 112 and/or a pharmacist associated with pharmacy 106 associated with prescription 110 to be filled, performs all required edit checks, prescription checks, and DURs. In one embodiment, prescriptions 110 are maintained by a pharmacist associated with a pharmacy 106 which is local to patient 104. A local pharmacy may comprise a pharmacy which is geographically close to patient 104, a pharmacy which is regularly used by patient 104 regardless of geographic distance and other suitable pharmacies with which patient has developed a relationship. In addition, if patient 104 changes pharmacies in order to stay with a particular pharmacist, the pharmacist may comprise the local pharmacy. After these checks have been completed satisfactorily, pharmacy system 112 considers the prescription ready to fill. Once all appropriate quality assurance procedures have been completed satisfactorily, at step 332, a technician or other user can initiate filling of the pending prescription request.

At step 334, pharmacy system 112 can determine whether the pending prescription request is to be adjudicated for payment by a third party. If so, at step 336, pharmacy system 112 transmits pertinent prescription information to an appropriate third party for payment and/or billing. For example, if the pending prescription request is to be funded by a federal program (e.g., indigent patient long-term care program), the pharmacy system can transmit the pending prescription information to the appropriate federal agency or the agency's intermediary for that program. In any event, the pending prescription request information can be conveyed to the third party by any appropriate communication medium (e.g., electronic transfer of digital files, etc.). If the pending prescription request is not to be adjudicated real-time for payment by a third party, pharmacy system 112 proceeds to step 344.

If the pending prescription information has been transmitted to a third party for adjudication, then at step 338, the pharmacy system 112 waits a predetermined amount of time for information from the third party to show that the claim for payment will be paid. If the claim will be paid within the predetermined period of time, pharmacy system 112 proceeds to step 344. Otherwise, if the claim will not be paid within the prescribed period of time, then at step 340, pharmacy system 112 creates an error message for a user or technician, which indicates that the claim will not be paid. Also, this message can indicate any possible errors in the prescription or claim information that may have prohibited payment of the claim. At step 342, a user or technician can correct error(s) in the prescription information, if any, and initiate reprocessing of the prescription request (step 330).

If the pending prescription request has been properly processed, at step 344, pharmacy system 112 initiates a procedure to fill the pending prescription request. For example, pharmacy system 112 can add a transaction record for processing the pending prescription request. This record can include a flag to indicate that the transaction has a "central fill" status, and can be filled by a central fill facility. At step 346, pharmacy system 112 adds the pending prescription request to a queue of transaction records for prescriptions waiting to be filled by central fill system 126. Central fill system 126 may alternatively be referred to as a central fill queue.

At step 348, pharmacy system 112 transmits the transaction real-time or otherwise to central fill system 126 via an appropriate communication link, such a non-proprietary link or proprietary link. In general, a suitable data or other communications link may be used. In one embodiment, transmitting information via the proprietary link may support status monitoring, data gathering, billing and other information gathering capabilities associated with the prescription request. Thus, checking, processing and determining of the formula to be prescribed may be done at pharmacy 106 local to patient 104 and prescription 110 may be maintained by the local pharmacy as well.

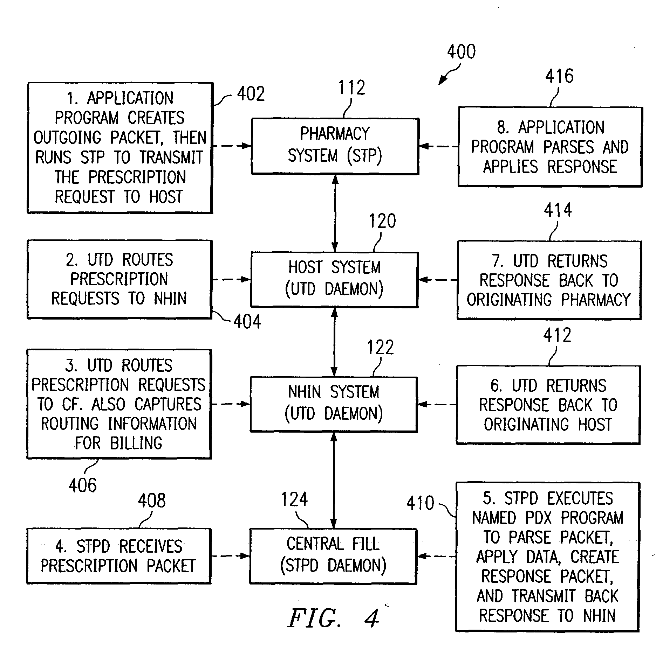

FIGURE 4 is a flow diagram that illustrates an exemplary method 400 which can be used to transmit prescription request information between pharmacy system 112 and central fill system 126. In one embodiment, the transmission of prescription request information may comprise a communications link based on a Transmission Control Protocol/Internet Protocol (TCP/IP) network and that prescription information can be transmitted from pharmacy system 112 to central fill system 126 in real-time. In one embodiment, real-time communication involves attempting to communicate information within a suitably short time frame after the information is provided; however, actual communication of the information may be delayed or even fail completely in one or more instances. For example, information may be communicated immediately after receipt of the information or may be sent on as the information is received. As such, referring to step 402 in FIGURE 4, pharmacy system 112 executes an application program that creates a packet of data containing prescription request information for transmission (e.g., packet formatted in accordance with TCP/IP). The application program then executes a Simple Transaction Processor (STP) communications program to transmit the packet containing the prescription request information to host system 120. Essentially, an STP program functions as a messaging hub, and can route addresses and other information (e.g., prescription data packets) throughout a network. An STP program can also be used to transmit status update requests, formulary update requests, etc. In one embodiment, if pharmacy system 112 is part of a group or chain of related pharmacies, host system 120 is located at a centralized site for the group or chain (e.g., chain headquarters).

At step 404, for this example embodiment, host system 120 routes (e.g., using a UTD) the prescription request information in data packet form to NHIN system 122. At step 406, NHIN system 122 routes the prescription request packet to central fill system 126. At step 408, an STP Daemon (STPD) included at central fill system 126 receives the prescription request packet from NHIN system 122. In addition to receiving prescription request packets, an STPD can also execute computer programs, and receive status update requests and formulary update requests.

In response to receiving a prescription request packet, at step 410, central fill system 126 (e.g., using an STPD) executes an application program to parse the packet and retrieve the prescription request information for further processing. In one embodiment, patient information, transmission number, prescription number, drug code, drug name, drug make and quantity to be filled are included within the packet. Also, central fill system 126 creates a packet with appropriate response information (e.g., acknowledges that a prescription request has been received), and transmits the response packet back to NHIN system 122 (e.g., using an STPD). At step 412, NHIN system 122 transmits the response packet to host system 120 (e.g., using a UTD). In return, at step 414, host system 120 transmits the response packet to pharmacy system 112 (e.g., using a UTD). At step 416, an application program at pharmacy system 112 parses the received response packet, and retrieves the response information for updating pharmacy system 112.

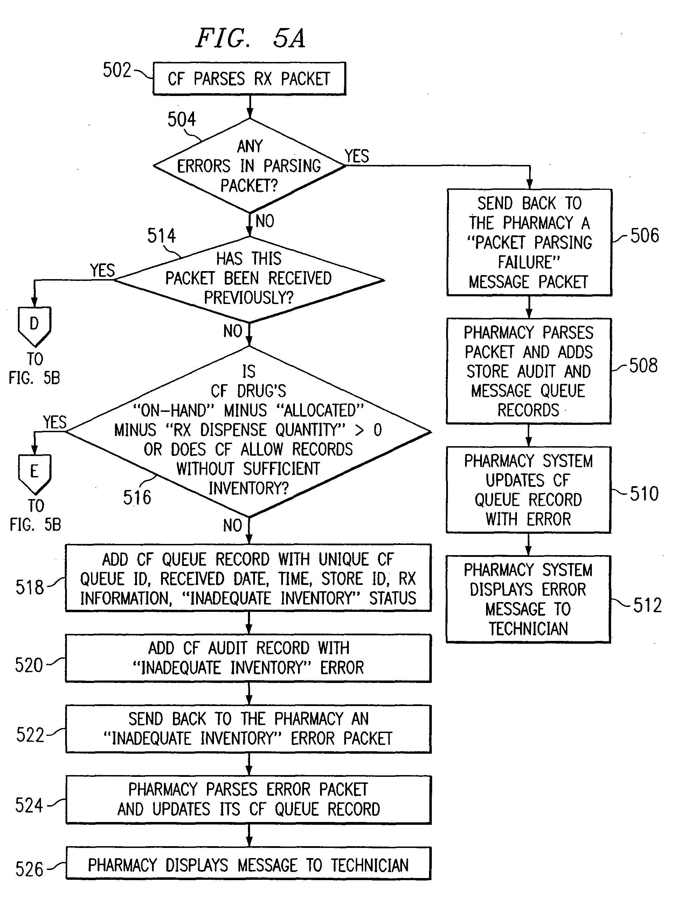

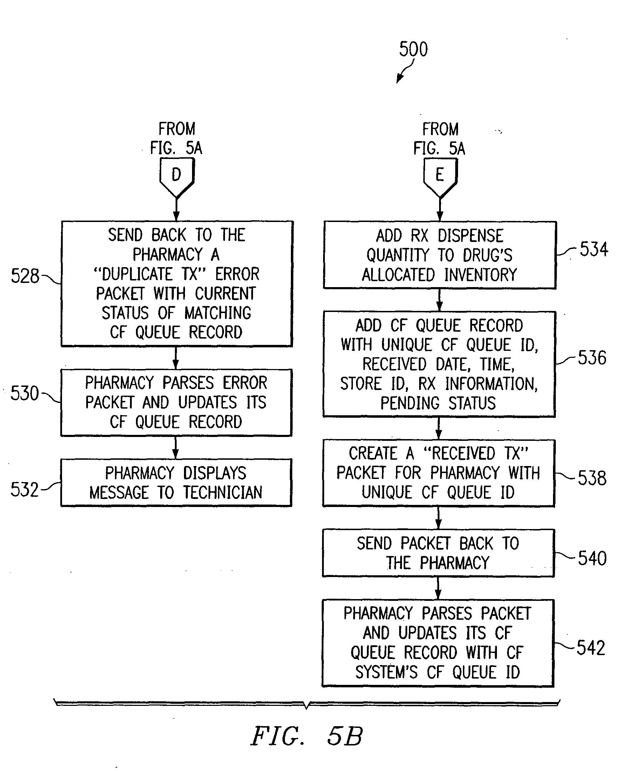

FIGURES 5A and 5B are a flow diagram that illustrates an exemplary computer-implemented method 500 for handling prescription filling according to one embodiment of the present invention. At step 502, central fill system 126 (using an application program) to parses a prescription request packet received from pharmacy system 112 via an appropriate communication link. At step 504, central fill system 126 determines whether there are any errors associated with the prescription request packet being parsed. If so, central fill system 126 creates a "packet parsing failure" message packet and transmits that packet back to pharmacy system 112 at step 506 (e.g., as a response packet illustrated by FIGURE 4).

If pharmacy system 112 receives a "packet parsing failure" message packet, at step 508, pharmacy system 112 parses the error message information from the received packet, and adds appropriate audit and message queue records to the error message information. Audit files can be maintained by pharmacy system 112 for pharmacy stores, in order to record errors and notifications to be reported to a pharmacy system administrator. At step 510, pharmacy system 112 updates its central fill queue record with the error message information. At step 512, pharmacy system 112 displays the error message information on a computer monitor to a user or technician.

Returning to step 504, if central fill system 126 determines that there are no errors in the prescription request packet being parsed, then at step 514, central fill system 126 determines whether this packet was previously received. For example, a unique identifier may be associated with each packet. If not, at step 516, central fill system 126 determines whether there is not enough inventory on-hand to fill this prescription request. In one embodiment, a home delivery prescription request can be set up so that the check for adequate inventory can be ignored or pushed forward. As a result, central fill system 126 is enabled to order the item(s) involved. For example, a home delivered prescription may not need to be delivered to the patient for three days, and the check for adequate inventory may be postponed or repeated at a later time while still allowing delivery to the patient within the three days. If there is not enough central fill inventory on-hand, at step 518, central fill system 126 adds a central fill queue record for the transaction record being processed. The added central fill queue record includes a unique central fill queue identifier, the date and time the prescription request was received, the identity of the local pharmacy in pharmacy system 112 that originated the prescription request, the prescription request information, and an "inadequate inventory" status flag. At step 520, central fill system 126 adds a central fill audit record that includes the "inadequate inventory" error. At step 522, central fill system 126 transmits an "inadequate inventory" packet back to pharmacy system 112 (e.g., as a response message illustrated by FIGURE 4). At step 524, pharmacy system 112 parses the received error packet and updates the central fill queue record accordingly to reflect the inadequate inventory error. At step 526, pharmacy system 112 displays an "inadequate inventory" error message for that prescription request to a user or technician (such as on a computer monitor).

Returning to step 514, if the prescription request packet had been previously received, then at step 528, central fill system 126 transmits a "duplicate transmission" error packet back to pharmacy system 112 (e.g., as a response packet shown in FIGURE 4). At step 530, pharmacy system 112 parses the received error packet and updates the central fill queue record accordingly. At step 532, pharmacy system 112 displays a "duplicate transmission" error message to a user or technician.

Returning to step 516, if central fill system 126 determines that there is enough inventory available on-hand to fill the prescription request, or if central fill system 126 is enabled to ignore inadequate inventory (e.g., home delivery prescription request), at step 534, central fill system 126 adds the quantity of the drug to be dispensed to the quantity shown in that drug's allocated inventory. At step 536, central fill system 126 adds a central fill queue record for the transaction record being processed. The added central fill queue record includes a unique central fill queue identifier, the date and time the prescription request was received, the identity of the local pharmacy in pharmacy system 112 that originated the prescription request, the prescription request information, and a "pending" status flag. At step 538, central fill system 126 creates a "received transaction" packet for transmission back to pharmacy system 112. The "received transaction" packet includes the unique central fill queue identifier for this prescription request. At step 540, central fill system 126 transmits the "received transaction" packet to pharmacy system 112 (e.g., as a response message shown in FIGURE 4). At step 542, pharmacy system 112 parses the "received transaction" packet, and updates the central fill queue record with the unique central fill queue identifier received from central fill system 126.

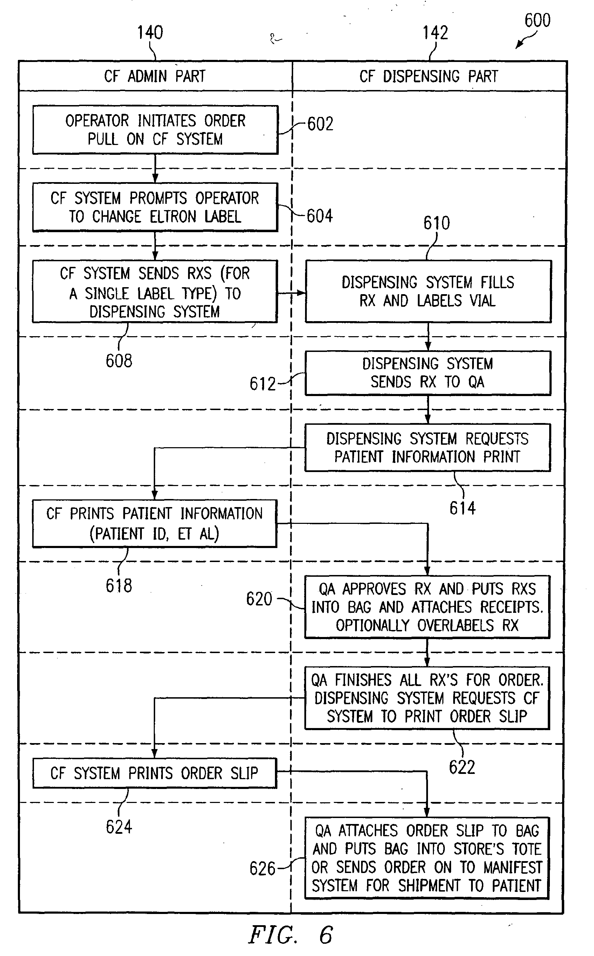

FIGURES 6A and 6B are a flow diagram illustrating a method 600 for filling prescriptions according to one embodiment of the present invention. In one embodiment, central fill system part 140 processes prescriptions to be transmitted to dispensing system part 142 in a batch mode. This procedure is referred to as an "order pull". At step 602, an operator associated with central fill system part 140 can initiate an "order pull" for a pending prescription request, by selecting an "Order Pull" option displayed (e.g., by a GUI) on a monitor and "pressing" a "start" function key. This procedure may also be automated to run at predetermined intervals throughout the day.

In response, central fill system part 140 updates the order identification field in the central fill queue to prepare the central fill queue with the pending transactions (orders to be filled) for transmission to dispensing system part 142. For example, a unique order ID is assigned to the associated central fill queue records, for each unique pharmacy NCPDP number and responsible party code. For this exemplary embodiment, a responsible party code is a pharmacy system code that can link multiple patients to a family. Also, an order can be one or more prescriptions grouped together by responsible party (e.g., usually for a family). Central fill system part 140 sorts the orders in the central fill queue by: label code -> store route group -> store stop - > store NCPDP number. If an order being processed is the final order for that day, as an option, an operator can select a range of order processing cut-off times. If such an option is selected, central fill system part 140 selects those pharmacy store routes for order processing with a central fill cut-off time that falls within the selected range. Central fill system part 140 then creates a data packet for each central fill queue record including the same order ID, and assigns the order packet a unique filename. In one embodiment, each order is associated with one or more related prescription requests (e.g., different prescriptions for two or more persons in the same family).

At step 604, central fill system part 140 prompts an operator (e.g., by displaying an appropriate message on a computer monitor) to change the label (e.g., bar-coded label), if necessary, to appropriately reflect the order to be filled. At step 608, central fill system part 140 transmits the order packet for each order (e.g., label type) to dispensing system part 142.

For example, central fill system part 140 can execute an STP program that transmits the order packet to a specified port at dispensing system part 142. If the STP program receives an acknowledgment ("ACK") message from dispensing system part 142, central fill system part 140 updates its prescription records with a "Sent" status flag. If the STP program does not receive an "ACK" message from dispensing system part 142, the order packet can be re-transmitted (up to a predetermined number of times).

Essentially, an "order pull" being processed can include many different orders. Each order can include one or more prescriptions for a responsible party. In one embodiment, central fill system part 140 transmits all of the prescription requests for one order at one time to dispensing system part 142. After transmitting an order packet to dispensing system part 142, central fill system part 140 waits for an acknowledgment message from dispensing system part 142. Upon receiving an acknowledgment message for an order packet, central fill system part 140 transmits the next order packet to dispensing system part 142. This procedure is repeated until all orders within that order pull have been transmitted to dispensing system part 142 and acknowledged.

At step 610, upon receiving an order packet from central fill system part 140, dispensing system part 142 parses the packet and applies the prescription order information received. In one embodiment, dispensing system part 142 dispenses only complete prescription drug(s) (i.e., no prescriptions are partially filled). In other words, if there is not enough stock on hand to fill a prescription request completely, dispensing system part 142 sends a response message (e.g., by executing an STP program) to central fill system part 140 that includes a "rejection" status flag for that prescription. For prescription requests that can be filled completely, dispensing system part 142 prints a generic, "stock label" for that prescription, and places the labeled prescription drug in the appropriate tote for shipping. Alternatively, as an option, dispensing system 142 can print a prescription label with the originating pharmacy's logo. All of the prescriptions in the order can be processed in the above-described way.

At step 612, a QA pharmacist associated with dispensing system part 142 can review each order being filled. For example, using a bar code scanner, a QA pharmacist can scan each prescription from an order. At step 614, dispensing system 142 sends an appropriate message (e.g., using an STP program) to central fill system part 140 which requests a printout of patient information associated with the scanned prescription. At step 618, central fill system part 140 sends an appropriate file for that scanned prescription to a printer, which can be accessed by the QA pharmacist involved. At step 620, the QA pharmacist retrieves the patient information from the printer, and double-checks the prescription information with the printout. If the review is successful, the QA pharmacist can approve the prescription for delivery. If the prescription is approved, the QA pharmacist places the prescription drug into a bag and attaches appropriate receipts to the bag. The QA pharmacist can place an updated prescription label on the bottle. This labeling is done to match the central fill bottle label with that of the retail pharmacy.

After the QA pharmacist has approved the prescription for shipping, dispensing system part 142 creates a status update packet and sends it to central fill system part 140. Upon receiving the status update packet, central fill system part 140 updates the central fill queue with the prescription information from the dispensing system part 142.

At step 622, when all of the prescriptions in an order have been processed (e.g., filled or rejected), dispensing system part 142 sends a message to central fill system part 140 that requests the central fill system part to print an order slip. An order slip is a document that lists each prescription in an order. At step 624, central fill system part 140 prints an order slip that includes all of the prescriptions in that order. At step 626, a pharmacist associated with dispensing system part 142 places all prescriptions and patient information related to an order into an order bag, and attaches the appropriate order slip to that order bag. The order bag is then routed to a dispatching area for shipping to pharmacy system 112 (e.g., prescription transport or shipping route represented by link 117), or to a manifest area (e.g., for home delivery prescriptions). For example, a QA pharmacist can place an order bag into a tote destined for delivery to a particular pharmacy store, or a tote destined for a manifest system in order to home deliver the order bag directly to the patient.

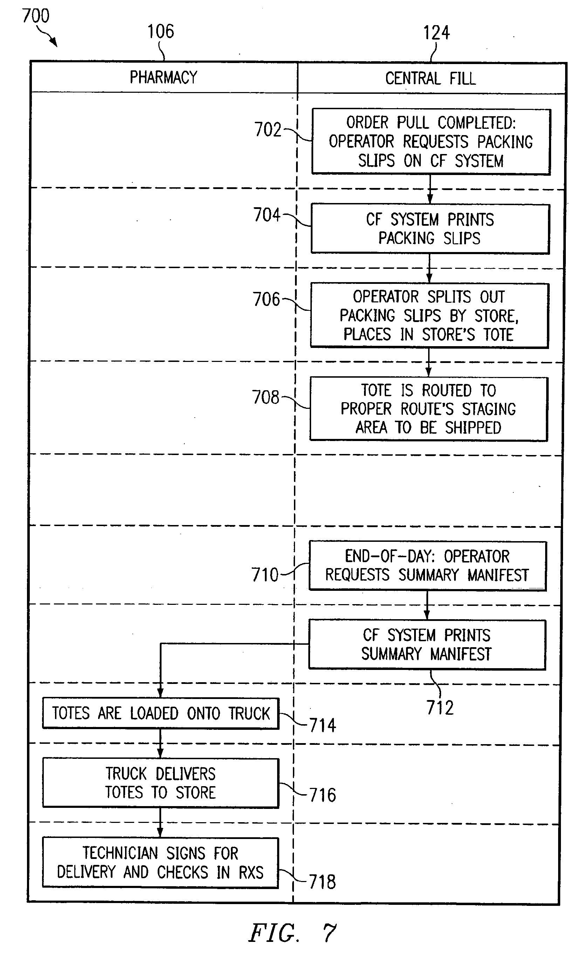

FIGURE 7 is a flow diagram illustrating a method 700 of operation of central fill system 126 according to one embodiment of the present invention. At step 702, when an operator has completed an order pull, and the order processing for that order pull has been completed, the operator can select the option "print packing slips" on a computer monitor associated with central fill system 126. The operator can then select (e.g., from a list of completed order pulls) which order pull is to have packing slips printed. A packing slip is a document that lists each prescription in a tote. The operator can then select the "start printing" function key. At step 704, in response to the operator's instructions, central fill system 126 prints packing slips for the selected order pulls.

When printing one or more packing slips for a selected order pull, central fill system 126 selects those prescriptions in the central fill queue that have been approved by the QA pharmacist for that particular order pull. The printed report can be sorted in order by route group -> route -> stop. Central fill system 126 then updates the status of those prescriptions in the central fill queue to "Ready for Shipment to Pharmacy". At step 706, an operator at central fill system 126 can separate the packing slips by store, place the appropriate packages with packing slips into the totes for the respective pharmacy stores, and seal the totes. At step 708, each tote is conveyed to an appropriate staging area for shipping to a pharmacy store.

At step 710, when all order processing has been completed for that day, and orders are ready to be shipped to the appropriate pharmacy stores, an operator can request central fill system 126 to print Summary Manifest documents (step 712). A Summary Manifest is a document that lists the stops on a shipping route and the items to be delivered at each stop. For these reports, central fill system 126 selects those prescriptions in the central fill queue that have a status of "Ready for Shipment to Pharmacy". After these prescriptions have been selected, central fill system 126 updates the status of these prescriptions in the central fill queue as "Shipped". At step 714, the totes can be loaded onto a truck for shipping. The truck driver can use the Summary Manifest for guidance in loading the truck and delivering the totes to the appropriate pharmacy stores. At step 716, the totes are delivered to the pharmacy stores. At step 718, a technician at pharmacy system 112 can sign the Summary Manifest and thereby acknowledge receipt of the shipped prescriptions. The technician at pharmacy system 112 then opens the tote and checks in the prescriptions filled at the central fill pharmacy by using a bar-code scanner. The pharmacy system will set those prescriptions to a status of "Processed," which means that the prescription has been placed in the bin and is ready for patient pick-up.

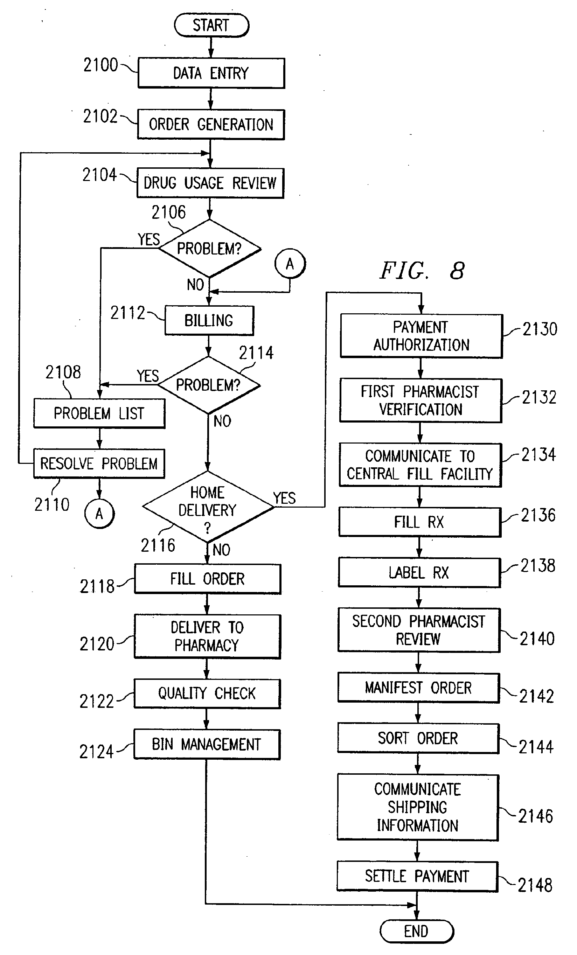

FIGURE 8 is a flowchart illustrating handling of orders 114 according to one embodiment of the present invention. The method begins at step 2100 where prescription information 200 associated with prescriptions 110 received at pharmacy 106 is entered into pharmacy system 112. For example, a technician or other personnel may enter data a computer associated with pharmacy system 112 from a handwritten prescription. Next, at step 2102, order 114 is generated by pharmacist 108. Pharmacist 108 may group one or more sets of prescription information 200 into order 114 using suitable criteria. Order 114 provides pharmacist 108 increased efficiency by supporting grouping of prescriptions 110. For example, grouping prescriptions into an order 114 may allow the pharmacist to more easily prescriptions between family members. Pharmacist 108 may also determine the priority associated with order 114. In one embodiment, generation of order 114 may involve determinations similar to those performed from steps 306 to 324 in FIGURES 3A and 3B

Pharmacist 108 determines shipping information 202 while generating order 114. More specifically, pharmacist 108 indicates whether the filled order 114 should be delivered back to pharmacy 106 associated with pharmacist 108 or to a different location. In one embodiment, delivering a filled order 114 to a location other than pharmacy 106 comprises home delivery. For example, filled orders 114 may be delivered to a patient's home or a managed care facility.

Then, at step 2104, pharmacist 108 performs a DUR on order 114 to check for possible medical problems with respect to patient 104. For example, pharmacist 108 may check for harmful drug interactions.

Proceeding to decisional step 2106, pharmacist 108 determines whether any problems are associated with order 114. A problem may comprise, for example, unrealistic or contradictory data in prescription information 200, or other problems associated with prescriptions 110. For another example, a prescription hand written by a physician may be illegible. If pharmacist 108 identifies one or problems, then the YES branch of decisional step 2106 leads to step 2108. At step 2108, the problem is added to problem list 118 for handling. Pharmacist 108 may optionally determine a priority for the problem. Then, at step 2110, pharmacist 108 or others resolve problems in problem list 118. For example, physicians 102 and/or patients 104 may be contacting regarding problems associated with orders 114. The method then returns to step 2104 where the DUR may be performed again to identify any further problems.

Returning to decisional step 2106, if problems are found with order 114, then the YES branch of decisional step 2106 leads to step 2112. At step 2112, billing information 206 associated with order 114 may be examined by pharmacist 108 for problems. Proceeding to decisional step 2114, if problems are found with billing information 206, then the YES branch of decisional step 2114 leads to steps 2108 and 2110 where the problems may be entered into problem list 118 and handled. Once the problems have been handled, the method returns to step 2112 where billing information 206 may be examined again for further problems. If no problems are found then the NO branch of decisional step 2116 leads to step 2116.

At decisional step 2116, pharmacy system 112 determines whether pharmacist 108 has indicated home delivery for order 114. In one embodiment, delivery to any location besides pharmacy 106 associated with pharmacist 108 comprises home delivery. If home delivery is not indicated, then the NO branch of decisional step 2116 leads to step 2118. At step 2118 central fill facility 124 fills order 114. More specifically, central fill facility 124 places requested pharmaceuticals into bottles or other suitable containers and places a label identifying the pharmaceutical onto the bottle or other container. Next, at step 2120, the filled order 114 is suitably packaged and delivered to pharmacy 106. Proceeding to step 2122, a quality check is performed by pharmacist 108 on the filled order 114. Pharmacist 108 may verify that the label and the actual pharmaceutical in the bottle are correct, and may perform other suitable quality assurance checks. Then, at step 2124, pharmacist 108 performs bin management. Bin management comprises organizing the one or more prescriptions 110 filled by order 114 so that the filled prescriptions may be distributed to patients 104. For example, filled prescriptions may be sorted alphabetically by the first letter of the patient's last name. The method then ends.

Returning to decisional step 2116, if home delivery has been indicated in shipping information 202, then the YES branch of decisional step 2116 leads to step 2130. At step 2130, payment authorization may be performed by pharmacist 108 on order 114. More specifically, pharmacist 108 determines whether order 114 can be paid for. For example, billing information 206 may be examined to determine how order 114 is to be paid. Then, pharmacist 108 may use pharmacy system 112 to generate an estimated order cost for order 114 and an estimated value for the shipping of order 114 based on shipping information 202. Based on the estimated cost for order 114 plus the cost of shipping order 114, pharmacist 108 may authorize a credit card to determine whether the credit card can cover the expected cost or may verify that sufficient funds are available to cover a check. Pharmacist 108 may also contact an insurer who may be paying all or a portion of the cost of order 114 to determine whether the prescriptions associated with order 114 will be covered.

Proceeding to step 2132, pharmacist 108 performs a first pharmacist verification on order 114. The first pharmacist verification comprises evaluating order 114 for correctness and medical issues. For example, pharmacist 108 may identify potential issues associated with pharmaceuticals in order 114 based on the pharmacist's historical relationship with patient 104. It should be noted that the checks performed in the first pharmacist verification may overlap portions of the checks made as part of the DUR. If pharmacist 108 detects problems or other issues with order 114, pharmacist 108 may use physician information 208, and/or patient information 204 to resolve the issues with patient 104 and physician 102. In one embodiment, pharmacist 108 may enter a problem into problem list 118 for resolution.

Once pharmacist 108 has been satisfied with order 114, pharmacy system 112 communicates order 114 to central fill facility 124 at step 2134. Order 114 may be communicated through host 120 and NHIN 122. Then, at step 2136, fill system 126 fills order 114 under control of master system 132. For example, master system 132 may separate out order 114 based on prescription information 200 for more efficient filling by fill system 126, such as via parallel filling across multiple robotic filling lines.

Next, at step 2158, master system 132 labels the filled prescriptions in order 114. Master system 132 may apply a suitable label to the container holding filled prescriptions in order 114. For example, master system 132 may generate a full color label with the name and logo of pharmacy 106 which owns prescriptions in order 114 along with information regarding the prescription itself.

Then, at step 2040, a pharmacist at central fill facility 124 performs a second pharmacist review. The second pharmacist review may comprise suitable verifications and checks on filled order 114 to those performed in the first pharmacies verification at step 2132. For example, the pharmacist at central fill facility 124 may verify that the proper pharmaceuticals and the proper dosages have been provided by fill system 126. And that the attached label is correct. The second pharmacist review may also involve other suitable verifications and checks.

Proceeding to step 2142, master system 132 hands filled order 114 to manifest system 134 for manifesting. Manifesting comprises packaging filled order 114 for shipment based on the shipping information of 202. For example, special packaging may be needed for liquid pharmaceuticals versus solid pharmaceuticals. Manifesting further comprises determining who and how filled order 114 will be shipped. For example, manifest system 134 may determine whether the U.S. Postal Service or UPS is more suitable for delivering order 114. Manifest system 134 may also determine the cost associated with shipping filled order 114 and use the cost information in selecting the shipper. Once manifest system 134 has determined the shipper and the cost, an appropriate label may be generated and applied to the shipping container holding filled order 114. Next, at step 2144, manifest system 134 sorts one or more orders of 114. More specifically, orders 114 may be sorted by shipper into various locations at central fill facility 124 for pickup by the appropriate shipper. For example, United Parcel Service packages may be separated from U.S. Postal Service packages. Then, at step 2146, shipping information at 202 is updated with shipping cost and shipper information as determined by manifest system 134 and communicated to master system 132. Master system 132 then communicates the updated shipping information 202 to pharmacy system 112 and may set the status for order 114 to "processed". In one embodiment, master system 132 communicates updated shipping information 202 to NHIN 122 for distribution through host 120 to pharmacy system 112.

Proceeding to step 2148, pharmacy system 112 finally settles payment for order 114. For example, based on billing information 206, a credit card may be finally charged or a check may be cashed. The method then ends.

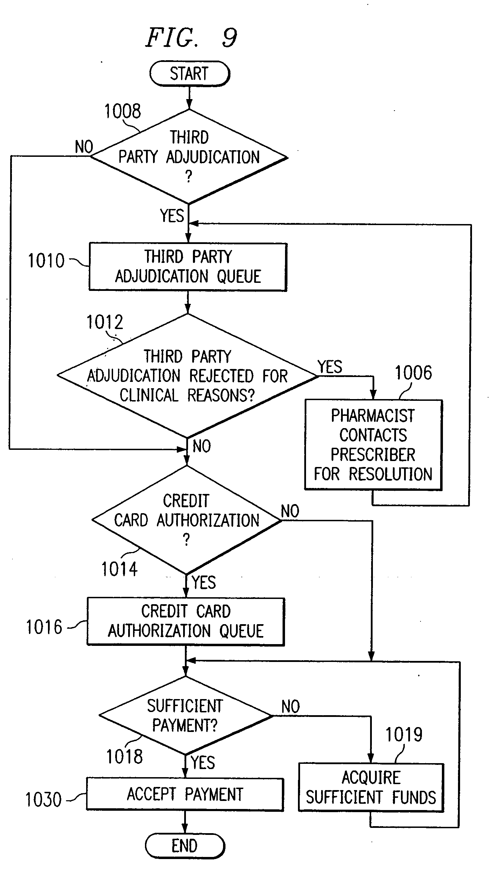

FIGURE 9 is a flowchart illustrating further details of payment processing for prescription 110 according to one embodiment of the present invention. In this embodiment, home delivery is from a central fill facility or other approved suppliers. In addition, in this embodiment, ownership of the prescription to be filled remains with the local pharmacy. For example, a patient may request home delivery of a filled prescription to the patient's residence independent of the pharmacy which owns the prescription.

The method begins at decisional step 1008 where pharmacy system 112 determines whether payment for prescription 110 involves third-party adjudication. For example, a government insurance program may be indicated as the payee for prescription 110. If order 114 involves third-party adjudication, then the YES branch of decisional step 1008 leads to step 1010. At step 1010, order 114 is added to a third-party adjudication queue. The third-party adjudication queue may comprise software and/or hardware in suitable combination operable to maintain a list of orders 114 to be adjudicated with one or more third-parties for payment. In addition, the third-party adjudication queue may maintain status and other suitable information associated with order 114 until payment is made. For example, status information regarding whether order 114 has been approved, denied or awaiting decision by the government or other insurer may be maintained. The third-party adjudication queue may track orders 114 which have been approved or denied in part. Proceeding to decisional step 1012, pharmacy system 112 determines whether third-party payment has been rejected for clinical reasons. If third-party payment has been rejected for clinical reasons, then the YES branch of decisional step 1012 leads to step 1006 where physician 102 is contacted regarding the rejection so appropriate action can be taken. If third-party payment is rejected, but not for clinical reasons, then the NO branch of decisional step 1012 leads to decisional step 1014.

Returning to decisional step 1008, if third-party adjudication is not involved with payment for prescription 110, then the NO branch of decisional step 1008 leads to decisional step 1014. At decisional step 1014, pharmacy system 112 determines whether a credit card is being used to pay for order 114 based on billing information 206. If credit card payment is to be used, then the YES branch of decisional step 1014 leads to step 1016. At step 1016, a request for authorization of a credit card associated with order 114 is added to a credit card authorization queue. The credit card authorization queue comprises software and/or hardware in suitable combination operable to maintain a list of one or more credit card requests for authorization of payment associated with orders 114. For example, the credit card authorization queue may comprise a portion of the software associated with pharmacy system 112. The credit card authorization queue may also support communication with credit card issuers for resolving authorizations and maintain status information associated with the credit card authorizations, such as allowed, pending or disallowed. In one embodiment, pharmacy system 112 may communicate credit card authorization requests to the credit card issuer via TCP/IP. If no credit card authorization is to be performed, then the NO branch of decisional step 1014 leads to decisional step 1018.

At decisional step 1018, pharmacy system 112 determines whether sufficient funds have been provided, authorized or are otherwise available to pay for order 114. For example, a credit card may authorize for only a portion of the cost of order 114. If sufficient funds have not been provided, authorized or otherwise made available to pay for order 114, then the NO branch of decisional step 1018 leads to step 1019. At step 1019 pharmacy system 112 acquires additional funds as appropriate to pay for order 114. For example, pharmacy system 112 may contact patient 104 and notify them of the lack of sufficient funds. Pharmacy system 112 may also accept the additional funds, such as checks, money orders, electronic funds transfers, credit cards and other suitable funds or methods of payment, to pay for order 114. Once sufficient funds are available to pay for order 114, the YES branch of decisional step 1018 leads to step 1030. At step 1030, pharmacy system 112 accepts payment for order 114. In one embodiment, a workflow engine may be used for handling home delivery payment processing and fill of a pending prescription may not be allowed until the workflow engine allows the fill after receiving payment or payment authorization for the pending prescription.

Although one or more embodiments of the method and apparatus of the present invention have been illustrated in the accompanying drawings and described in the foregoing detailed description, it will be understood that the invention is not limited to the one or more embodiments disclosed, but is capable of numerous rearrangements, modifications and substitutions without departing from the spirit and scope of the invention as set forth by the following claims.

## Claims

1. A system for filling prescriptions comprising:
software stored on a computer readable medium and operable to:
receive an order from a pharmacy, the order having a shipping location and at least one set of prescription information;
generate at least one prescription label based on the order;
determine a shipping cost associated with the order based on the shipping location;
determine a cost for the order based on the shipping cost and the order; and
ship the order to the shipping location.

2. The system according to Claim 1, wherein the software is further operable to:
dispense a pharmaceutical into a container based on the prescription information;
apply the prescription label to the container.

3. The system according to Claim 2, wherein the software is further operable to:
package the containers associated with the order into a package;
generate a shipping label based on the order;
apply the shipping label to the package; and
wherein shipping the order comprises the software further operable to ship the package to the shipping location.

4. A method for filling prescriptions comprising:
receiving an order from a pharmacy, the order having a shipping location and at least one set of prescription information;
generating at least one prescription label based on the order;
determining a shipping cost associated with the order based on the shipping location;
determining a cost for the order based on the shipping cost and the order;
shipping the order to the shipping location.

5. The method according to Claim 4 and further comprising:
dispensing a pharmaceutical into a container based on the prescription information;
applying the prescription label to the container.

6. The method according to Claim 5 and further comprising:
packaging the containers associated with the order into a package;
generating a shipping label based on the order;
applying the shipping label to the package; and
wherein shipping the order comprises shipping the package to the shipping location.

7. The system or method according to any preceding claim, wherein the prescription label is customized based on a pharmacy associated with the order.

8. The system according to any preceding system claim, wherein the software is further operable to communicate the cost to the pharmacy, or the method according to any preceding method claim further comprising communicating the cost to the pharmacy.

9. The system according to any preceding system claim, wherein determining the shipping cost comprises the software further operable to:
determine a shipper cost for at least one shipper;
select the shipper for the order based on the shipper cost; and
determine a handling cost associated with the order, or the method according to any preceding method claim wherein determining the shipping costs comprises:
determining a shipper cost for at least one shipper;
selecting the shipper for the order based on the shipper cost; and
determining a handling cost associated with the order.

10. The system according to any preceding system claim, wherein determining the cost comprises the software further operable to:
determine a total cost for the order based on a price associated with each pharmaceutical to be dispensed; and
generate the cost based on the sum of the shipping cost and the total cost; or the method according to any preceding method claim wherein determining the cost comprises:
determining a total cost for the order based on price associated with each pharmaceutical to be dispensed; and
generating the cost based on the sum of the shipping cost and the total cost.

11. A system for prescription management comprising:
software stored on a computer readable medium and operable to:
receive at least one prescription at a pharmacy;
generate an order associated with at least one of the prescriptions;
determine a shipping location associated with the order;
communicate the order to a central fill facility for filling the prescriptions associated with the order;
receive a cost associated with the order from the central fill facility, the cost including a shipping cost and an order cost; and
settle payment for the order based on the cost.

12. A method for prescription management comprising:
receiving at least one prescription at a pharmacy;
generating an order associated with at least one of the prescriptions;
determining a shipping location associated with the order;
communicating the order to a central fill facility for filling the prescriptions associated with the order;
receiving a cost associated with the order from the central fill facility, the cost including a shipping cost and an order cost; and
settling payment for the order based on the cost.

13. The system according to Claim 11, wherein the software is further operable to:
determine, at the pharmacy system, an estimated cost associated with the order; and
authorize, at the pharmacy system, payment based on the estimated cost; or the method according to claim 12 wherein it further comprises:
determining, at the pharmacy system, an estimated cost associated with the order; and
authorizing, at the pharmacy system, payment based on the estimated cost.

14. The system according to Claim 11 or Claim 13, wherein the software is further operable to:
determine an estimated cost associated with the order, the estimated cost including an estimated order cost based on the prescriptions associated with the order and an estimated shipping cost based on the shipping location associated with the order; and
determine whether sufficient funds are available to pay for the order based on the estimated cost and billing information associated with the order; or the method of Claim 12 or Claim 13 further comprising:
determining an estimated cost associated with the order, the estimated cost including an estimated order cost based on the prescriptions associated with the order and an estimated shipping cost based on the shipping location associated with the order; and
determining whether sufficient funds are available to pay for the order based on the estimated cost and billing information associated with the order.

15. The system according to Claim 14, wherein determining whether sufficient funds are available comprises the software further operable to authorize a credit card for the estimated cost when the billing information indicates that the credit card will be used to pay for the order; or the method according to Claim 14 wherein determining whether sufficient funds are available comprises authorizing a credit card for the estimated cost when the billing information indicates that the credit card will be used to pay for the order.

16. The system according to Claim 14 or Claim 15, wherein determining whether sufficient funds are available comprises the software further operable to determine whether sufficient funds are available for a check when the billing information indicates that the check will be used to pay for the order; or the method according to Claim 14 or Claim 15 wherein determining whether sufficient funds are available comprises determining whether sufficient funds are available for a check when the billing information indicates that the check will be used to pay for the order.

17. The system according to any one of system claims 11 to 16, wherein the software is further operable to:
enter a set of prescription information respectively associated with each prescription received at the pharmacy into a pharmacy system associated with the pharmacy;
determine whether the prescription information has been properly entered; and
perform a drug utilization review on the order; or the method of any one of method claims 12 to 16 further comprising:
entering a set of prescription information respectively associated with each prescription received at the pharmacy into a pharmacy system associated with the pharmacy;
determining whether the prescription information has been properly entered; and
performing a drug utilization review on the order.

18. The system or method according to Claim 17, wherein the order is associated with the respective sets of prescription information associated with the prescriptions associated with the order.

19. The system according to any one of system Claims 11 to 18, wherein generating the order comprises the software further operable to:
select at least one of the prescriptions for the order; and
associate the selected prescriptions with the order; or the method of any one of method Claims 12 to 18 wherein generating the order comprises:
selecting at least one of the prescriptions for the order; and
associating the selected prescriptions with the order.

20. The system or method according to Claim 19, wherein the selected prescriptions are for a single family.

21. The system or method according to Claim 19, wherein the selected prescriptions are for a plurality of distinct patients who reside at a managed care facility.

22. The system according to any one of system Claims 11 to 21, wherein determining the shipping location comprises the software further operable to:
select the pharmacy as the shipping location when the order is to be shipped to the pharmacy after the order has been filled; and
select a home delivery location based on the order when the order is to be shipped to a location associated with a patient associated with at least one of the prescriptions associated with the order after the order has been filled; or the method of any one of method Claims 12 to 21 wherein determining the shipping location comprises:
selecting the pharmacy as the shipping location when the order is to be shipped to the pharmacy after the order has been filled; and
selecting a home delivery location based on the order when the order is to be shipped to a location associated with a patient associated with at least one of the prescriptions associated with the order after the order has been filled.

23. The system according to Claim 22, wherein determining the shipping location further comprises the software further operable to determine whether a state associated with the shipping location allows home delivery of the order; or the method according to Claim 22 wherein determining the shipping location further comprises determining whether a state associated with the shipping location allows home delivery of the order.

24. The system according to any one of system Claims 11 to 23, wherein settling payment for the order comprises the software further operable to:
determine at least one payment technique associated with the order; and
charge the payment techniques for the order based on the payment technique; or the method of any one of Claims 12 to 23 wherein settling payment for the order comprises:
determining at least one payment technique associated with the order; and
charging the payment techniques for the order based on the payment technique.

25. The system or method according to Claim 24, wherein the payment technique comprises a credit card.

26. The system or method according to claim 24 or Claim 25, wherein the payment technique comprises a third-party payor.

27. A system for prescription management comprising:
means for receiving at least one prescription at a pharmacy;
means for generating an order associated with at least one of the prescriptions;
means for determining a shipping location associated with the order;
means for communicating the order to a central fill facility for filling the prescriptions associated with the order;
means for receiving a cost associated with the order from the central fill facility, the cost including a shipping cost and an order cost; and
means for settling payment for the order based on the cost.

28. A method for processing prescription requests, comprising the steps of:
receiving a plurality of prescription requests;
creating a queue of prescription requests from the received plurality of prescription requests, each prescription request in the queue eligible to be filled by a central fill inventory;
filling a prescription request in the queue of prescription requests from the central fill inventory;
dispensing at least one drug from the central fill inventory;
generating a mailing label including mailing address information associated with an intended recipient of the at least one drug; and
affixing the mailing label to a package for mailing, the package for the dispensed at least one drug.

29. The method of Claim 28 and further comprising processing a claim for payment for the at least one prescription request at a billing subsystem.

30. A system for prescription fulfillment, comprising:
means for receiving a prescription of a prescribed drug for a patient at a pharmacy designated by the patient; and
means for maintaining the prescription at the designated pharmacy, while having the prescription filled with the prescribed drug at a remote third-party facility for delivery of the drug to a location designated by the patient on behalf of but independent of the designated pharmacy.

31. A method for prescription fulfillment, comprising:
receiving a prescription of a prescribed drug for a patient at a pharmacy designated by the patient; and
while maintaining the prescription at the designated pharmacy, having the prescription filled with the prescribed drug at a remote third-party facility for delivery of the drug to a location designated by the patient on behalf of but independent of the designated pharmacy.

32. The system of Claim 30, or the method of Claim 31, wherein the location designated by the parties comprises a residential address of the patient.

33. The system of Claim 30 or Claim 32, further comprising means for having a refill of the prescription filled with the prescribed drug at the remote third-party facility for delivery of the prescribed drug directly to the address of the patient; or the method of Claim 31 or Claim 32 further comprising having a refill of the prescription filled with the prescribed drug at the remote third-party facility for delivery of the prescribed drug directly to the address of the patient.

34. The system of or method of any one of Claims 30 to 33, wherein the pharmacy maintains ownership of the prescription.

35. The system or method according to any one of Claims 30 to 34, wherein the patient orders the refill through the designated pharmacy.

36. The system or method of Claim 35, wherein the patient orders the refill electronically through the designated pharmacy.

37. The system or method of Claim 36, wherein the patient orders the refill through an Internet site associated with the designated pharmacy.

38. The system of any one of Claims 30 to 37, further comprising means for collecting payment for filling the prescription at the designated pharmacy; or the method of any one of Claims 31 to 37 further comprising collecting payment for filling the prescription at the designated pharmacy.

39. The system of any one of Claims 30 to 37, further comprising means for collecting payment information electronically from the patient; or the method of any one of Claims 31 to 37 further comprising collecting payment information electronically from the patient.

40. A system for prescription fulfillment at a centralized facility, comprising:
means for receiving at a third-party centralized site an order for a drug prescribed to a patient in a prescription maintained at a pharmacy designated by the patient;
means for filling the prescription with the prescribed drug at the third-party centralized site; and
means for providing the drug for shipment to an address specified by the patient independent of the designated pharmacy.

41. A method for prescription fulfillment at a centralized facility, comprising:
receiving at a third-party centralized site an order for a drug prescribed to a patient in a prescription maintained at a pharmacy designated by the patient;
filling the prescription with the prescribed drug at the third-party centralized site; and
providing the drug for shipment to an address specified by the patient independent of the designated pharmacy.

42. The system of Claim 40 or the method of Claim 41, wherein the address comprises a residential address of the patient.

43. The system of Claim 40 or Claim 42, further comprising means for shipping the prescribed drug directly to a residence of the patient; or the method of Claim 41 or Claim 42 further comprising shipping the prescribed drug directly to a residence of the patient.

44. A system for filling prescriptions comprising:
means for receiving an order from a pharmacy, the order having a shipping location and at least one set of prescription information;
means for generating at least one prescription label based on the order;
means for determining a shipping cost associated with the order based on the shipping location;
means for determining a cost for the order based on the shipping cost and the order;
means for shipping the order to the shipping location.

45. A system for processing prescription requests, comprising:
means for receiving a plurality of prescription requests;
means for creating a queue of prescription requests from the received plurality of prescription requests, each prescription request in the queue eligible to be filled by a central fill inventory;
means for filling a prescription request in the queue of prescription requests from the central fill inventory;
means for dispensing at least one drug from the central fill inventory:
means for generating a mailing label including mailing address information associated with an intended recipient of the at least one drug; and
means for affixing the mailing label to a package for mailing, the package for the dispensed at least one drug.

46. Software for processing prescription requests, the software embodied in a computer-readable medium and when executed operable to:
receive a plurality of prescription requests;
create a queue of prescription requests from the received plurality of prescription requests, each prescription request in the queue eligible to be filled by a central fill inventory;
fill a prescription request in the queue of prescription requests from the central fill inventory;
dispense at least one drug from the central fill inventory:
generate a mailing label including mailing address information associated with an intended recipient of the at least one drug; and affix the mailing label to a package for mailing, the package for the dispensed at least one drug.
